# EUROPEAN PATENT APPLICATION

(11) **EP 3 040 070 A1**
(43) Date of publication of application: **06.07.2016**
(21) Application number: 15202185.3
(22) Date of filing: 22.12.2015
(51) Int. Cl.: A61K 31/16, A61K 31/20, A61K 31/201, A61K 35/747, A61K 48/00, A61P 3/00, A61P 3/04, A61P 3/06, A61P 3/10, A61P 29/00, A61P 1/00

(54) **DIETARY METHODS USING LACTOBACILLUS PARACASEI SUBSP. PARACASEI F19 AS NAPE-PLD GENE CARRIER FOR PRODUCING ON DEMAND PEA OR OEA AND RELATIVE BIOLOGICAL DIETARY COMPOSITIONS THEREOF**

(30) Priority: 29.12.2014 IT RM20140757
(71) Applicant: Farmagens Health Care Srl, 00191 Rome (IT)
(72) Inventor: Sannetti, Augusto, I-00191 Rome (IT); Cuomo, Rosario, I-00191 Rome (IT); Sarnelli, Giovanni, I-00191 Rome (IT); Esposito, Giuseppe, I-00191 Rome (IT)
(74) Representative: Sarpi, Maurizio

(57) **Abstract**

Method enabling to modulate and customize the production of active lipids involved in exerting an antagonistic action to local inflammation and in modulating the mast cell reactivity, comprising the administering to an host the probiotic *Lactobacillus paracasei* subsp. Paracasei F19, genetically modified with a nucleotide vector comprising a nucleic acid encoding the human N-acyl-phosphatidylethanolamine phospholipase D (NAPE-PLD), in association with lipid substrates and precursors, to influence the enzymatic reactions of said probiotic agent in the host. The method is useful in the treatment of inflammatory bowel disorder and in the treatment of metabolic syndrome and for controlling of obesity.

SEQUENCE LISTING

<110> FARMAGENS HEALTHCARE Srl

<120> DIETARY METHODS USING LACTOBACILLUS PARACASEI SUBSP. PARACASEI F19 AS
NAPE-PLD GENE CARRIER FOR PRODUCING ON DEMAND PEA OR OEA AND RELATIVE
BIOLOGICAL DIETARY COMPOSITIONS THEREOF

<130> 9208

<150> ITRM2014A000757
<151> 2014-12-29

<160> 1

<170> BiSSAP 1.3

<210> 1
<211> 393
<212> PRT
<213> Homo sapiens

<400> 1
Met Asp Glu Asn Glu Ser Asn Gln Ser Leu Met Thr Ser Ser Gln Tyr
1               5                   10                  15
Pro Lys Glu Ala Val Arg Lys Arg Gln Asn Ser Ala Arg Asn Ser Gly
            20                  25                  30
Ala Ser Asp Ser Ser Arg Phe Ser Arg Lys Ser Phe Lys Leu Asp Tyr
        35                  40                  45
Arg Leu Glu Glu Asp Val Thr Lys Ser Lys Lys Gly Lys Asp Gly Arg
    50                  55                  60
Phe Val Asn Pro Trp Pro Thr Trp Lys Asn Pro Ser Ile Pro Asn Val
65                  70                  75                  80
Leu Arg Trp Leu Ile Met Glu Lys Asp His Ser Ser Val Pro Ser Ser
                85                  90                  95
Lys Glu Glu Leu Asp Lys Glu Leu Pro Val Leu Lys Pro Tyr Phe Ile
            100                 105                 110
Thr Asn Pro Glu Glu Ala Gly Val Arg Glu Ala Gly Leu Arg Val Thr
        115                 120                 125
Trp Leu Gly His Ala Thr Val Met Val Glu Met Asp Glu Leu Ile Phe
    130                 135                 140
Leu Thr Asp Pro Ile Phe Ser Ser Arg Ala Ser Pro Ser Gln Tyr Met
145                 150                 155                 160
Gly Pro Lys Arg Phe Arg Arg Ser Pro Cys Thr Ile Ser Glu Leu Pro
                165                 170                 175
Pro Ile Asp Ala Val Leu Ile Ser His Asn His Tyr Asp His Leu Asp
            180                 185                 190
Tyr Asn Ser Val Ile Ala Leu Asn Glu Arg Phe Gly Asn Glu Leu Arg
        195                 200                 205
Trp Phe Val Pro Leu Gly Leu Leu Asp Trp Met Gln Lys Cys Gly Cys
    210                 215                 220
Glu Asn Val Ile Glu Leu Asp Trp Trp Glu Glu Asn Cys Val Pro Gly
225                 230                 235                 240
His Asp Lys Val Thr Phe Val Phe Thr Pro Ser Gln His Trp Cys Lys
                245                 250                 255
Arg Thr Leu Met Asp Asp Asn Lys Val Leu Trp Gly Ser Trp Ser Val
            260                 265                 270
Leu Gly Pro Trp Asn Arg Phe Phe Phe Ala Gly Asp Thr Gly Tyr Cys
        275                 280                 285
Pro Ala Phe Glu Glu Ile Gly Lys Arg Phe Gly Pro Phe Asp Leu Ala
    290                 295                 300
Ala Ile Pro Ile Gly Ala Tyr Glu Pro Arg Trp Phe Met Lys Tyr Gln
305                 310                 315                 320

His Val Asp Pro Glu Glu Ala Val Arg Ile His Thr Asp Val Gln Thr
                325                 330                 335
Lys Lys Ser Met Ala Ile His Trp Gly Thr Phe Ala Leu Ala Asn Glu
            340                 345                 350
His Tyr Leu Glu Pro Pro Val Lys Leu Asn Glu Ala Leu Glu Arg Tyr
        355                 360                 365
Gly Leu Asn Ala Glu Asp Phe Phe Val Leu Lys His Gly Glu Ser Arg
    370                 375                 380
Tyr Leu Asn Asn Asp Asp Glu Asn Phe
385                 390

## Description

### Technical field

The present invention relates to the field of dietary food and clinical nutrition. In particular, the invention relates to dietary plans, customized and adaptable, supporting pharmacobiotic strategies by using probiotics, or their metabolites, or genetically engineered bacteria to amplify the benefits from the use of the probiotic, addressing the metabolic synthesis of endogenous substances (aliamidi) by the probiotic organisms based on the actual individual needs.

### Background of the invention

It is known that the composition of the intestinal micro biota of an individual is a significant determinant of the susceptibility to metabolic diseases, such as diabetes, obesity, cardiovascular diseases and chronic and degenerative inflammatory conditions. Appropriate action on intestinal micro biota can improve the course of these metabolic diseases. This has led to the development of strategies to reshape the set of symbiotic microorganisms of the human digestive tract in order to also include genetically modified bacteria expressing therapeutic factors protecting against the onset of specific related pathological conditions.

The N-acyl-phosphatidyl-ethanolamines (NAPE) are the precursors of a family of lipids, the N-acyl-ethanolamines (NAE), normally produced in the proximal part of the small intestine in response to food intake. The anorectic effect of these molecules exerting their action on three receptors: PPAR alpha, TRPV1 and GPR119, causing reduction of food intake, delayed gastric emptying, inhibition of lipid and increased oxidation of fatty acids has been demonstrated. A lipid rich diet suppresses the production of NAE resulting in a loss of their beneficial effects when the body mostly needs it. This led the researchers to speculate that the administration of specifically engineered bacteria to produce NAPE could compensate for this deficit. After synthesis, the molecule of NAPE is rapidly subjected to hydrolysis by phospholipase D, which converts it into its active form NAE.

The international patent application WO 2013/043719 relates to a method for treating obesity, insulin sensitivity and related conditions, which involves the administration of a bacterium genetically modified to overexpress and secrete the N-acyl-phosphatidyl ethanolamines (NAPE) and/or N-acyl ethanolamines (NAE) in a suitable vehicle.

In some embodiments of the invention the bacterium is a biosafety level 1 microorganism able to colonize the human intestine; in some embodiments of the invention the bacterium is selected from the genera Bacillus, Bifidobacterium, Bacteroidetes, Lactobacillus, Lactococcus, Enterobacteriaceae, Escherichium. In some embodiments the bacterium belongs to the species *E*. *coli,* preferably the probiotic *E. coli Nissle* (ECN). The bacterium is transformed by a vector comprising a nucleic acid molecule encoding an enzyme involved in the synthesis process of NAPE and/or NAE, for example NAPE acyltransferase or NAPE-phospholipase D (NAPE-PLD). The experimental results presented in support of the invention show that the intake of genetically modified bacteria to produce high amounts of NAPE in the intestinal micro biota represents a promising new therapeutic approach in the case of forms of prolonged treatment for a chronic condition, or obesity. According to the proposed approach, the patient is relieved from the necessity of a continuous daily administration frequency of the therapeutic compound. The long-term efficacy of the major clinical therapeutic protocols for obesity imposing a regulated lifestyle, is poor because of the difficulty in maintaining it for long periods of time.

The introduction of pNAPE-ECN, the probiotic *E. coli Nissle* 1917 transformed with the vector bearing the sequence of N-acyltransferase catalyzing the synthesis of NAPE in the intestinal flora of mice consuming a diet rich in fat, is able to determine a steady decrease of weight gain in mice up to six weeks after the bacteria administration. The treated animals retain the total body weight and adiposity lower than that of control animals even 12 weeks after the administration has stopped. This shows that taking a functional bacterium overexpressing NAPE is an effective strategy to ensure long-term treatment. Also repeated dosing at intervals of 4, 6 or 12 weeks of probiotic engineered represent a treatment regimen significantly easier than drugs that must be taken daily or a strict caloric restriction.

Although oral administration of *E. coli Nissle* has been shown to improve the course of inflammatory bowel disease, the molecular basis of its therapeutic effectiveness are not yet known [1-2]. Despite its clinical use, in fact, the genetic determinants of the probiotic features of *E. coli Nissle* are still little known. Recent studies have shown that *E. coli Nissle* bears a cluster of genes 'pks' islands that can produce a genotoxic hybrid peptide, the colibactina [3], which in vitro has been shown to be able of producing genetic damage, instability and genetic mutations in the mammalian allele [4]. According to what is reported, it is therefore clear that even the engineering of *E. coli Nissle* aimed to the hyperproduction of NAPE-PLD carries a potential genotoxicity risk of inherent in the bacterium itself that precludes safe use in Humans. However, it is noteworthy that the overexpression of NAPE enzyme in the probiotic, although providing positive effects in terms of release of precursors of NAE promoting the production of anorectic acting molecules, although working well in animal model, in a clinical perspective applicable to a broad spectrum of pathological conditions of the human being, the method is affected by the restriction of lipid production, too general and non-specific, that not always suits to the specific requirements of the various metabolic diseases.

A number of disadvantages and possible negative side effects do not recommend, or limit, the use of the solution proposed in WO 2013/043719 in many clinical or chronic conditions. In fact, the use of probiotic genetically modified microorganisms is not authorized by many international medical health organizations. In addition, the engineering of probiotic micro-organisms which has not been demonstrated and documented genetic stability thereof, especially if used for long time in chronic patients, can have serious adverse side effects, as documented by many publications and clinical trials. In this case, the major documented side effects are: bacterial translocation, pro-inflammatory effects, increased resistance to antibiotics, adverse effects on the metabolism, in particular lipid, uncontrolled immune stimulation, particularly negative on immunocompromised patients. Therefore, in order to optimize the therapeutic efficacy of the approach that uses engineered probiotic it would be useful to intervene by directing the enzyme substrate provided with a proper diet. The disadvantage intrinsically present in the simple overproduction of the NAPE enzyme, according to the teachings of the patent application WO 2013/043719, could be overcome by a strong integration between the enzymatic and nutraceutical activity, exploiting, depending on the case, the enzyme as a factory of pharmacologically active lipids in the intestine supported by a specific and suitable dietary protocol.

### Summary of the Invention

The method according to the present invention allows addressing "on demand" the enzymatic activity of NAPE-PLD depending on the state of health of the patient by providing a nutritional scheme responsive to the therapeutic and metabolic needs of the subject. In fact, such needs can be very different, for example in the case of Crohn's disease, obesity, ulcerative colitis, metabolic syndrome, etc ... A nonspecific production of NAPE-PDL in a patient with inflammatory bowel disease may also have anorectic effects, and potentially can preclude the benefits thereof or, worsen the patient's condition. Conversely, a diet specific for disease or group of diseases, able to address the metabolic activity of the bacteria *Lactobacillus paracasei* subsp. paracasei F19, acting as genetically stable carrier of NAPE, provides the possibility to achieve a clinical modulation and, moreover a therapeutic customization, hence increasing the effectiveness for the benefit of the patient [5-6]. Instead of an indiscriminate increase in NAPE, according to the teaching of WO 2013/043719, the method according to the present invention provides the administration of *Lactobacillus paracasei* subsp. paracasei F19, carrying the gene coding for NAPE-PLD, associated with a specific food supplementation that allows to obtain, depending on the addition of palmitic acid, or a salt thereof, or oleic acid, or a salt thereof, high levels of intestinal OEA or PEA, respectively exerting primarily an anti-inflammatory/analgesic action or calories intake control.

Such early, modular and selective "on demand" action is of fundamental importance since a generalized overproduction of NAPE, and consequently of yielded lipids, could determine contrasting actions, while addressing "a *priori"* and "on demand" the lipid type to be produced in the intestine, modulated by diet, allows to optimize the therapeutic effect. A further object of the invention are also the biological dietary compositions comprising the engineered *Lactobacillus paracasei* subsp. paracasei F19, carrying the gene coding for NAPE-PLD, of the dietary regimen that optimize the therapeutic effectiveness of the method.

### Description of the figures

In the figures:
- Figure 1 schematically shows the experimental plan;
- Figure 2 shows the release of PEA *in vitro* induced by LP-^{NAPE-PLD+} and LP-^{empty vector} (10¹⁰ cfu) in the presence of palmitic acid (10⁻¹⁰-10⁻⁷ M), 6, 12 and 24 hours after the induction.
- Figure 3 shows the effect in CD-1 mice of the administration of the composition comprising LP-^{NAPE-PLD+} in association with a daily dietary support of exogenous palmitate (from 0.003 to 0.03 g/ml/gavage) in reference to the parameters considered; Figure 3A shows the effect on the Disease Activity Index (DAI); Figure 3B shows the effect on nitrite release; Figure 3C shows the effect on iNOS expression level; Figure 3D shows the effect on COX-2 expression level; Figure 3E shows the effect on TNF**α** release; Figure 3F shows the effect on IL-1β**.** release.
- Figure 4 shows the histological analysis in a murine model of acute colitis induced by DSS treated by the method according to the invention;
- Figure 5 shows the amount of the infiltrating macrophages in sections of the murine model of acute colitis induced by DSS treated with the method according to the invention;
- Figure 6 shows the effect of the administration of the composition comprising LP-^{NAPE-PLD+} in association with a daily dietary support of exogenous palmitate (from 0.003 to 0.03 g/ ml/gavage) in IL-10 KO mice in reference to the parameters considered; Figure 6A shows the effect on the disease course (scale DAI); Figure 6B shows the effect on the nitrite release; Figure 6C shows the effect on the iNOS expression level; Figure 6D shows the effect on the COX-2 expression level; Figure 6E shows the effect on IL-1**β** release; Figure 6F shows the effect on TNFα**;**
- Figure 7 shows the effect of the administration of the composition comprising LP-^{NAPE-PLD+} in association with a daily dietary support of exogenous palmitate (from 0.003 to 0.03 g/ml) in colon biopsies from patients affected from ulcerative colitis in reference the considered parameters; Figure 7A shows the effect on nitrite release; Figure 7B shows the effect on iNOS expression level; Figure 7C shows the effect on COX-2 expression level; Figure 7D shows the effect on IL-1**β** release; Figure 7E shows the effect on TNF**α** release.
- Figure 8 shows histological analysis (by toluidine bleu) on duodenal biopsies of dyspeptic patients exposed to acid stimulation after daily administration of LP-^{NAPE-PLD+} or LP-^{empty vector} in the presence or absence of a co-administration of palmitate (0.03 to 0.3 g/ml).
- Figure 9 shows the amount of the considered parameters on duodenal biopsies sections from dyspeptic patients exposed to acid stimulation; Figure 9A shows the number of mast cells per mm² of tissue; Fig. 9B shows the extent of histamine release, Fig. 9C shows the tryptase level, Fig. 9D shows the protein TRPV1 expression level and Fig. 9E the TRPV4 expression level.
- Figure 10 shows the release *in vitro* of OEA induced by LP^{-NAPE-PLD+} and LP-^{empty vector} (10¹⁰ cfu) in the presence of oleic acid (10⁻¹⁰-10⁻⁷ M) 6, 12 and 24 hours after the induction;
- Figure 11 shows the effect of administration of the composition comprising LP-^{NAPE-PLD+} in association with a daily dietary support of exogenous oleate 0.03 g/ml/gavage to ob/ob mice in reference to the considered parameters; Figure 11A shows the food intake; Figure 11B shows the effect on the animal weight; Figure 11C shows the effect on the of cholesterol and triglycerides level in blood samples.

### Detailed Description of the invention

A first aspect of the invention consists in a method allowing to modulate and customize the production of pharmacologically active lipids by a specific diet in patients by the probiotic *Lactobacillus paracasei* subsp. paracasei F19, engineered to produce and secrete PEA and/or OA based on the clinical needs of the person the treatment is intended for in combination with lipid substrates and precursors able to influence the course of the enzymatic reaction of said probiotic.

The method according to the invention uses *Lactobacillus paracasei* subsp. paracasei F19 as a vehicle of probiotic intestinal production of PEA and OEA on-demand driven by nutraceutical supplementation, respectively, on the basis of palmitic acid, or a salt thereof, or oleic acid, or a salt thereof.

The possibility of producing PEA and OEA on demand is therefore aimed at creating a new therapeutic approach lactobacillus-mediated and nutraceutically integrated which can find very broad clinical use in all those diseases wherein such aliamides can exert a beneficial effect.

With the term aliamides it is intended a family of biological molecules, which PEA and OEA belong to, able to locally control the tissue mis-reactive state, by acting, via the receptor, along with the control systems expressed on the cell membrane of mast cells. In fact, aliamides, interacting with a mast cell receptor, block degranulation thereof, preventing the triggering of the phenomena basing cutaneous hyperreactivity and proinflammatory states. In some conditions mast cells are able to overexpress response to agonists stimuli, through the release of proinflammatory macromolecules (i.e. cytokines, vasoactive amines, proteolytic enzymes, bradykinin, histamine).

Aliamides are able to exert an antagonistic action to local inflammation, modulating the mast cell reactivity.

Palmitoylethanolamide (PEA) is an endogenous molecule that physiologically acts as a balancer in the processes associated with neuroinflammation, it is a cell physiological constituent.

The PEA also has cannabinergic effects. The cannabinergic effect should be due to its ability to act as a substrate for FAAH (fatty acid amide hydrolase), thereby reducing the release of arachidonic acid and, therefore, the synthesis of prostaglandins (anti-inflammatory effect).

As such, the PEA can act as a functional food, or more precisely, it is a dietary food for special medical purposes. In this case the integration of PEA feeding can be especially significant in the pathologies associated with conditions characterized by an inflammatory state.

Oleoylethanolamide (OEA) is the endogenous lipid produced by the small intestine during the meal, mediating the satiety signal; by feeding cells of the intestinal mucosa are stimulated to produce oleoylethanolamide acting as a messenger for the lipid satiety as a sort of hunger switcher, encouraging the feeding cessation, through the activation of the of peroxisomal proliferation receptors (PPAR).

The method according to the invention combines and exploits the beneficial and well-known properties of probiotics with those of an appropriate nutraceutical supplementation. The vehicle for probiotic intestinal production of PEA and OEA on-demand driven by nutraceutical dietary supplementation is *Lactobacillus paracasei* subsp. paracasei F19, which has been shown to have a inherent genetic stability, fundamental feature that makes it unique among probiotics and identifies it as ideal carrier of natural molecules with beneficial effects on health (nutraceuticals). The probiotic bacterium *Lactobacillus paracasei* subsp. paracasei F19 was isolated from human small intestine, it is very stable in variable pH conditions and it is not degraded by stomach acid and bile secretions (US Pat. No. US 6,599,504). In addition, unlike other probiotic, *L. paracasei* subsp. paracasei F19 is able to ferment most carbohydrates, including lactose, galactose, tagatose, galacto-oligosaccharides, oligosaccharides, lactulose and all the carbohydrates present in the milk and dairy products. The method according to the invention is intended to subjects, preferably humans and non-human primates and any other organism, that can benefit from the use of the method according to the present description. This person can also be referred to as patient, individual, subject, host, user, and organism. In a particularly preferred embodiment of the invention the recipient subject of the method is a human being. According to another aspect of the invention specific nutritional plans for different types or categories of individuals as a function of the disease or disorder diagnosed according to the clinical and biochemical techniques usually followed in medical practice are provided.

Various foods, sometimes suitably enriched with functional ingredients and nutraceuticals are the source of nutrients.

Thus the present invention provides a simple and accurate method to determine, in subjects using therapeutic programs based on the administration of NAE and NAPE by engineered probiotics, the right amount of lipid substrates and nutritional supplements to ensure maximum effectiveness of the therapeutic program taking into account all factors affecting the final outcome. It is known that males and females differ in their lipid metabolism due to different sex hormone pattern and different gene expression; furthermore changes in hormonal equilibrium can result in changes of lipid requirements with age. It should be also considered that macronutrients and lipids molecules are very sensitive to oxidative stress, one of the contributory causes of aging. Therefore, a synergistic and controlled use of antioxidant agents, as well as minerals, phytochemical agents, vitamins, contributes to the final effect of the formulations according to the invention.

Since according to the invention the customized nutritional program integrates the method using the probiotic inducing a high level of NAE and NAPE in the intestinal microflora of the host, the modulation of the final effect is exerted by adjusting the type of lipid substrates and precursors supplied by feeding to influence the course of metabolic reactions in the subject.

Thus, for example, among the many types of acylammides that can be taken by feeding, a diet enriched in palmitic acid, or palmitate, in the intestine produce predominantly N- palmitoylethanolamide, the amide of palmitic acid (16:0) and ethanolamine, while a diet rich in particular in oleic acid, or a salt thereof, will provide the amide of oleic acid (18:1) and ethanolamine, N-oleoyletanolamide as substrate for the NAPE-AT and NAPE-PLD enzymes.

The field of application of the invention herein described are the treatment of inflammatory bowel disease (Crohn's disease and ulcerative colitis), functional gastrointestinal syndromes characterized by chronic pain (functional dyspepsia and irritable bowel syndrome) and/or diseases associated with overweight and obesity such as cardiovascular disease, metabolic syndrome, diabetes. Therefore, according to the invention a method for prescribing and providing formulations of nutritional supplements for patients according to an appropriate scheme is provided. Thus, the physician selects the appropriate formulation of the functional food or nutritional supplement and instructs the patient about how to take the formulation; the patient obtained the formulation of the nutritional supplement follows the instructions given by the physician.

The method according to the invention involves the administration of a composition comprising the bacterium *Lactobacillus paracasei* subsp. paracasei F19 genetically modified to achieve the overproduction of N-acyl phosphatidylethanolamide (NAPE) and/or of N-acylethanolamminide (NAE), saturated and/or unsaturated fatty acids, or salts thereof, any further nutraceutical elements, and a edible vehicle, suitable for the oral administration of the composition itself. Object of the present invention is also the bacterium *Lactobacillus paracasei* subsp. paracasei F19 transformed with a nucleotide vector comprising a nucleic acid encoding the human N-acyl-phosphatidylethanolamine phospholipase D (NAPE-PLD) of sequence SEQ. ID NO: 1.

The probiotic bacterium L. *paracasei* subsp. paracasei F19 was used to overproduce N-acyl phosphatidylethanolamine (NAPE) and/or N-acylethanolamine (NAE) transformed by a vector comprising a nucleic acid encoding a polypeptide, or enzyme, for the production of NAPE and/or NAE (accession number: Q6IQ20) (full lenght). The polypeptide, or enzyme, for the production of NAPE and/or NAE is the human N-acyl-phosphatidylethanolamine phospholipase D (NAPE-PLD) of sequence of SEQ ID NO: 1, which hydrolyses the N-acyl-phosphatidylethanolamine (NAPE) producing N-acylethanolamine (NAE) and phosphatidic acid. In a particularly preferred embodiment the probiotic bacterium is engineered in lyophilized form in amounts varying between 1x10⁹ and 1x10¹¹ cfu (colony forming units) per milligram of composition. The bacterium engineered according to the invention is part of a probiotic/nutraceutical composition, also part of the invention, administered according to the method and comprising fatty acids, or their salts, in an amount ranging from 3 to 9 mg per 100 mg of composition.

According to the invention the fatty acids predominantly are oleic acid and palmitic acid. In one embodiment the method according to the invention the is used for the treatment of inflammatory intestinal disorders, such as inflammatory bowel disease (IBD), Crohn's disease and ulcerative colitis, and functional gastrointestinal syndromes characterized by chronic pain, such as functional dyspepsia and irritable bowel syndrome. It provides the assumption of the composition comprising the probiotic *Lactobacillus paracasei* subsp. paracasei F19 ^{NAPE-PLD+} in combination with palmitic acid, or a salt thereof, wherein palmitic acid, or its salt, is present in variable amount between 3 and 9 mg per 100 mg of composition.

In another embodiment the method according to the invention is used for the treatment of metabolic syndrome and in the control of obesity. It provides the intake of a composition comprising the probiotic *Lactobacillus paracasei* subsp. paracasei F19 ^{NAPE-PLD+} in combination with oleic acid, or a salt thereof, wherein the oleic acid, or its salts, is present in variable amount between 3 and 9 mg per 100 mg of composition. The nutraceutical composition administered by the method of the invention may further comprise dietary fiber, maltodextrin, glucoligosaccharides, fructooligosaccharides (GOS/FOS), and prebiotic agents, pectin, inulin, amino acids, minerals and vitamins, especially vitamin C, soy or corn proteins, antioxidant agents, commonly used in food compositions in variable amount in the final nutraceutical composition.

In some embodiments the composition comprising the L. *paracasei* subsp. paracasei F19^{NAPE-PLD+} is provided in an edible vehicle suitable for oral administration to the recipient of the treatment. The composition according to the invention is provided in an edible product suitable for oral administration, examples of edible product suitable for oral administration include, but are not limited to: diet bars, beverages, juices, milk, functional beverages, beverages containing dietary fiber, food, yogurt, candy, gum, capsules, granulated powder, tablets. It is shown below, solely for purposes of description and not of limitation, two examples of nutraceutical composition of the present invention.

**EXAMPLE 1 - Nutraceutical biologic composition for use in the treatment of inflammatory bowel disease:**

| Ingredient | Amount per 100 g of product | Daily Dose |
|---|---|---|
| *L. paracasei* F19 NAPE PLD+ | 1200-2400*10⁹ (cfu) | 12-24*109 (cfu) |
| Palmitic acid | 3-9 g | 300-900 mg |

**EXAMPLE 2 - Nutraceutical biologic composition for use in the treatment of obesity:**

| Ingredient | Amount per 100 g of product | Daily Dose |
|---|---|---|
| *L. paracasei* F19 NAPE PLD+ | 1200-2400*10⁹ (cfu) | 12-24*109 (cfu) |
| Oleic acid | 3-9 g | 300-900 mg |

Additional ingredients in the formulation may vary according to the packaging customized for patient or disease.

The dosing regimen of the formulation in support of the method according to the invention is defined by the physician according to the real need of the subject recipient of the treatment. Such a need is determined by clinical and biochemical analysis which the subject is subjected to before starting treatment and at regular intervals during treatment to verify the effectiveness of the method, the good tolerability by the subject and the lack of side effects. The amount of palmitate and oleate administered to the patient with the formulation ranges from 0.03 to 0.3 micrograms per mg of preparation. The dosing regimen is changed and optimized according to the clinical effectiveness in the subject. In particular, for IBD the disease course is assessed through the evaluation of clinical parameters, such as reduction of diarrhea and increase in consistency of the stool, pain reduction and normalization of body temperature, and biochemical parameters, such as leukocytosis and inflammatory markers of fecal calprotectin reduction, etc. For functional gastrointestinal disorders (dyspepsia and irritable bowel syndrome) the reduction of the severity of specific symptoms and abdominal pain are evaluated.

The effectiveness of the method that involves the administration of the specific biologic formulation enriched in oleic acid according to the invention, the trend of obesity and associated disorders is monitored by means of evaluation of the body weight and body mass index (BMI) reduction and by periodic review of blood glucose and insulin, total cholesterol, HDL fraction/LDL, triglycerides, blood pressure reduction and normalization of transaminases (AST and ALT).

### EXPERIMENTAL PART

### EXAMPLES

The invention will now be illustrated with reference to examples and methods of use and experimental assays not limiting the scope of application of the invention. Study 1 - Biological/nutraceutical composition in combination with a diet enriched in palmitic acid for producing PEA *on demand* for the use in the treatment of inflammatory bowel disease (Crohn's disease and ulcerative colitis).
1. The effect of the method according to the invention that involves taking a biological/nutraceutical composition comprising *Lactobacillus paracasei* NAPE-PLD+ (LP-^{NAPE-PLD+)} has been preliminarily evaluated in mouse models of acute and/or chronic experimental colitis and, subsequently, the effect of the composition was assessed in the organotypic cultures isolated from human patients suffering from ulcerative colitis and/or Crohn's disease.
   Bacteria LP-^{NAPE-PLD+} and LP-^{NAPE-PLD-} (empty vector) (control bacterium carrying the empty pNIC-BASY plasmid) were cultured *in vitro* and exposed to increasing concentrations of palmitic acid (10⁻¹⁰-10⁻⁷ M). At different time intervals (6-12-24 hours post incubation) an amount of bacterial supernatant was collected and subjected to analysis for the quantification of the released PEA.
   The efficacy of the LP-^{NAPE-PLD+} was evaluated by observing the improvement of clinical and biochemical parameters in an experimental protocol of acute colitis induced by dextran sodium sulfate (DSS) in 7 days.
   In the above experimental procedure, the combined effect of a diet with palmitic acid (0.03 to 0.003 g /ml/gavage) and 10¹⁰ cfu of LP-^{NAPE-PLD+} (or LP-^{NAPE-PLD-} depending on the experimental plan) provided by gavage was assessed by analyzing the clinical and biochemical improvement of the induced colitis by Disease Activity Index; DAI score and by histopathology and biomolecular investigation considering the macrophage infiltration of colon and the cytokines expression (TNF**α**, IL-1**β**) and inflammatory proteins (iNOS, COX-2).
2. According to the obtained results the efficacy of LP-^{NAPE-PLD+} with the addition of palmitic acid was evaluated in the animal model of chronic colitis of IL-10 KO mice, which genetically develop a chronic colitis similar to human chronic colitis. Efficacy assessment was done after 21 days by evaluating the macrophage infiltration in the colon and the expression of cytokines (TNF**α,** IL-1**β,** etc) and inflammatory proteins (iNOS, COX-2, etc).
3. The effectiveness of the method according to the invention which involves the intake of LP-^{NAPE-PLD+} in association with palmitic acid in the culture medium was evaluated in organotypic cultures of human biopsies derived from subjects suffering from ulcerative colitis. Also in this case it was evaluated the release of pro-inflammatory cytokines (TNF**α,** IL-1β) and of specific proteins (iNOS, COX-2).

### Experimental method

### Measuring PEA release in vitro

The supernatant from LP-^{NAPE-PLD+} or LP-^{empty vector} cultures in the presence of external support of palmitic acid (10⁻¹⁰-10⁻⁷ M) in 6-12-24 hours was analyzed by liquid chromatography coupled to spectrometry mass (LC-MS/MS) using a mass spectrometer 325 triple quadrupole LC-MS/MS (Agilent Technologies Italy, Cernusco s/N, Italy). The PEA levels were quantified and expressed in concentration (nM).

### Animals

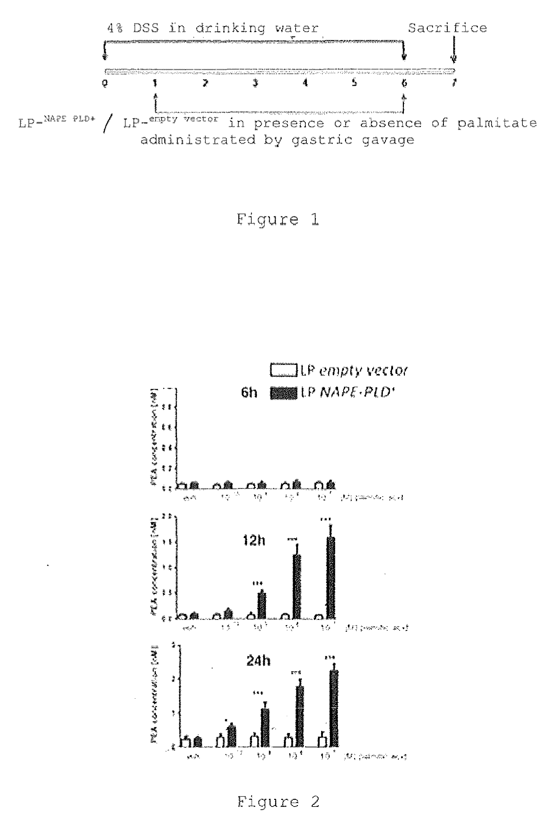

In the study 6 week old mice, CD-1 strain (25-35 g) were used. The animals were randomly divided into three experimental groups, each consisting of n = 10 animals according to the following scheme: a) control group; b) group with colitis; c) group with experimental colitis receiving LP^{NAPE-PLD+} or ^{LP-empty vector} in the presence or absence of external palmitate dietary support 0.003 to 0.3 mg/ml, administered in the volume of 1 ml per day by gastric gavage. The experimental colitis was induced by the administration of DSS (4% w/v, MW 36000-50000) in the drinking water of the animals for 6 consecutive days, starting from day 1 as described in the experimental design shown in Figure 1. Six weeks old mice IL-10 KO strain, which spontaneously develop colitis, were used to confirm the preliminary data obtained in the model of acute colitis by DSS. In this case, the mice were treated according to the experimental design with LP-^{PLD NAPE +} or LP-^{empty vector} and the effect of the daily administration of the biological/nutraceutical preparation with palmitate (from 0.003 to 0.030 mg/ml) administered in the volume of 1 ml per day by gastric gavage was assessed as described above.

### Activity index of disease: Disease Activity Index (DAI)

The severity degree of the disease was expressed by DAI scale, based on the evaluation of several parameters characterizing the experimental colitis, both in its induction phase and progression. The weight of the animal, the presence of blood in the stool and stool consistency have been used as reference of colitis symptoms for 7 days; a score scale from 0 to 12 depending on the parameters: weight loss/rectal bleeding/diarrhea/stool consistency was established.

### Organotypic cultures of human colon

Colon biopsies were obtained from patients suffering from ulcerative colitis. All patients giving biopsy sample gave their informed consent to the economic exploitation through patenting of the results of the study and invention that ensued. Biopsies were cultured in Dulbecco's Modified Eagle Medium (DMEM) with FBS at 37 °C in 5% CO₂ atmosphere of 95% O₂.

Depending on the experimental protocol performed in the presence/absence of (10¹⁰ cfu) LP NAPE-^{PLD+} or LP^{empty vector}, in the presence or absence of supplementation with palmitic acid (0.003 to 0.03 mg/ml), the release of pro-inflammatory mediators was assessed.

### Protein extraction and western blot analysis.

Cytosolic extracts from colon biopsies were obtained by homogenization in iced hypotonic lysis buffer. The extracts were subjected to electrophoresis through a 12% polyacrylamide gel. Proteins were transferred onto nitrocellulose membrane saturated with no-fat dry milk, and then incubated with a anti-rabbit iNOS antibody (Santa Cruz Biotech, California, USA), anti-rabbit COX-2 antibody (Santa Cruz Biotech, California, USA), anti-mouse (β-actin antibody (Santa Cruz Biotechnology, Santa Cruz, California, USA). The membranes were incubated with specific horseradish peroxidase conjugated antibodies (HRP) (Dako, Milan, Italy). Immune complexes were revealed by chemiluminelescence (Amersham Biosciences, Milan, Italy). The blots were analyzed by scanning densitometry (GS-700 Imaging densitometer; Bio-Rad). The results were expressed as OD (arbitrary units for mm²) and normalized with respect to the (β-actin expression level.

### TNFα, IL-1β and nitrites dosage

The immunoenzymatic assay (ELISA) for IL1**α** and TNF**β** (Abcam, Cambridge, UK) was conducted according to the protocol provided by the manufacturer. The absorbance was measured using a micro plate reader Titertek (BioRad, Milan, Italy) and the levels of released cytokines were quantified with respect to specific standard curves. For the determination of nitrite released by colon homogenates from animals and humans Griess assay was used.

The preliminary results of the study that used a biological/nutraceutical composition according to the invention in combination with diet enriched in palmitic acid have shown that the addition of exogenous palmitate, at increasing doses (10⁻¹⁰-10⁻⁷ M) determines an significant and time-dependent increase of PEA production *in vitro* induced by LP ^{NAPE-PLD+} LP^{empty vector} compared to the control, as shown in Figure 2. Figure 2 shows the time-dependent trend of the release PEA in LP-^{NAPE-PLD +} and LP-^{empty vector} (10¹⁰ cfu) in the presence of palmitic acid *in vitro* (10⁻¹⁰-10⁻⁷ M) (6-12-24 hours). The results are representative of four experiments in triplicate. (*p<0.05; and ***p<0.001 vs. LP-^{empty vector}, respectively).

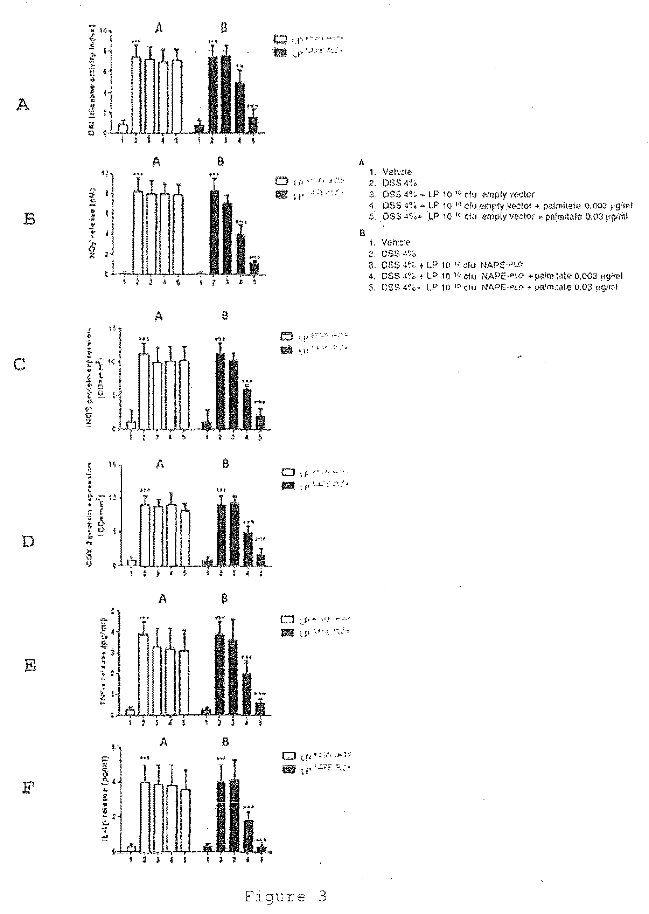

In addition, as shown in Figure 3, administration of LP-^{NAPE-PLD+} in association with a daily dietary support of exogenous palmitate (0.003 to 0.03 g/ml/gavage) in CD-1 mice results in a significant improvement of the following parameters compared to administration of the same composition with the control LP^{empty-vector}_{:}
(A) by symptoms: through clear reduction of the disease course and improvement of disease activity;
(B) molecular: through a significant reduction of inflammatory parameters (inhibition of nitrite release, expression level of iNOS and COX-2, expression level of IL-1**β** and TNF**α;**
(C) microscopic/tissue: reduction of the macrophages infiltrate in the mucosa) in CD-1 mice exposed to experimental colitis induced by 4% dextran.

Figure 3 shows the effect of daily administration of LP-^{NAPE-PLD+} or LP^{empty vector} in the presence/absence of a co-administration of palmitate (0.03 to 0.3 mg/ml/gavage) respectively on the disease activity index (DAI) (Fig. 3A), on the release of nitrite (Fig. 3B), on the expression level of iNOS (Fig. 3C), the expression level of COX-2 (Fig. 3D) and the TNF**α** (Fig. 3D) and IL-1**β** (Fig. 3E) release. Results are representative of n = 4 experiments in triplicate. (*P<0.05; and ***P<0.001 vs. LP empty vector, respectively). *** P<0.001 vs. vehicle; *P<0.05; ***P <0.001 vs. DSS respectively.

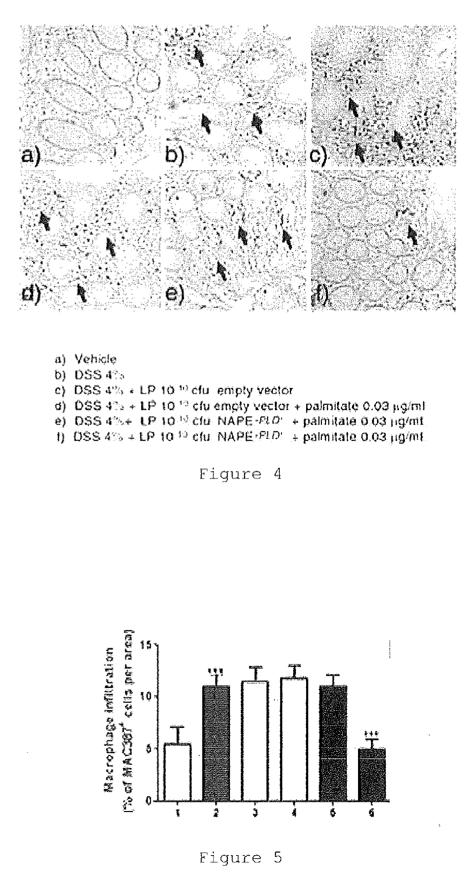

Figure 4 shows the effect of the daily administration of the probiotic LP-^{NAPE-PLD+} or LP-^{empty vector} (10¹⁰ cfu) in the presence/absence of a co-administration of palmitate (0.03 to 0.3 mg/ml/gavage) on macrophage infiltration in the colon of CD-1 mice treated with DSS (arrows indicate infiltrating macrophages and detected by MAC387 antibody. (20X magnification).

Figure 5 shows the quantization of the infiltrating macrophages (***P<0.001 vs. vehicle; ***P<0.001 vs. DSS respectively; the results are representative of n = 3 experiments in triplicate).

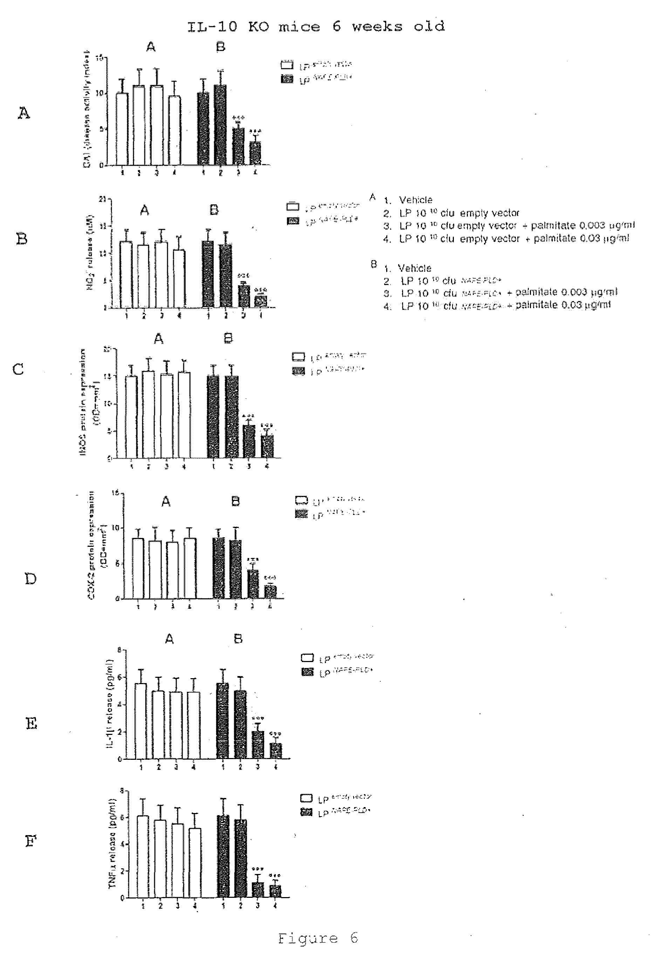

These data were then also confirmed in an experimental model of chronic colitis in IL-10 KO mice and in colon biopsies from patients suffering from ulcerative colitis. The preliminary data are shown in Figure 6 demonstrating that, in IL-10 KO mice, daily administration (for 2 weeks) of LP^{NAPE-PLD+} associated with dietary support with palmitate from 0.003 to 0.03 g/ml determines: (A) considerable reduction of the parameters of the course of disease severity (scale DAI) (Fig. 6A); (B) reduction of colon inflammation parameters with a significant reduction of nitrites (Fig. 6B), iNOS (Fig. 6C), COX-2 (Fig. 6D), IL-1**β** (Fig. 6E) and TNF**α** (Fig. 6F) levels.

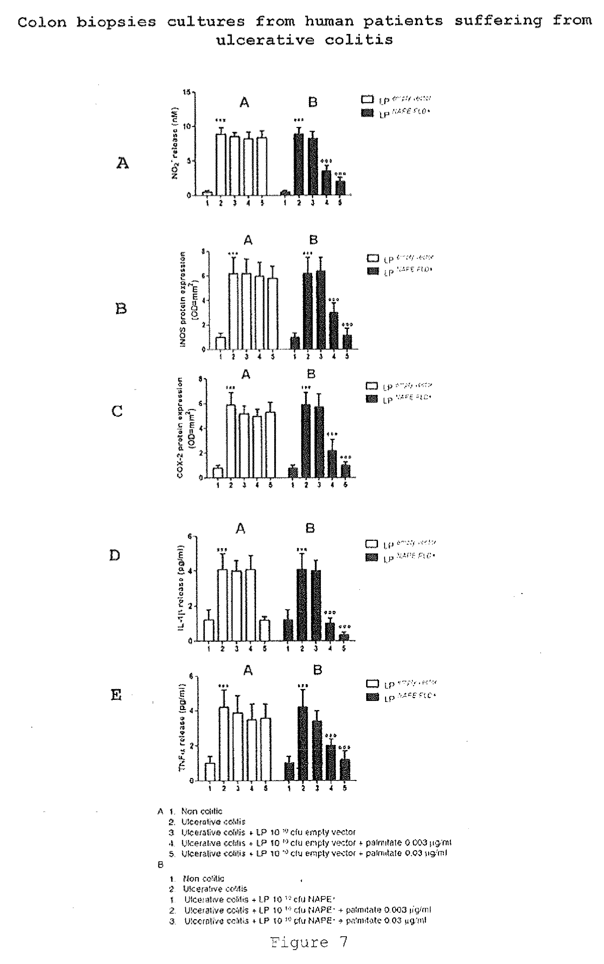

Colon biopsies from patients suffering from ulcerative colitis with LP^{NAPE-PLD+} associated with palmitate 0.003 to 0.03 g/ml has shown, *in vitro,* a significant reduction of inflammation indicators, through a dose-dependent effect depending on the substrate of exogenous palmitate administered (0.003 to 0.03 g/ml) (Figure 7).

The effect of LP^{NAPE-PLD+} or LP^{empty vector} incubation with supplementation with palmitate (0.03 to 0.3 mg/ml) is shown respectively through: evaluation of the nitrite release (Fig. 7A), expression of iNOS (Fig. 7B), expression of COX-2 (Fig. 7C), IL-1β (Fig. 7D), and TNF**α** (Fig. 7E) release, and in mucosal samples derived from human colitis colon or control. ***P <0.001 vs. vehicle; ***P<0.001 vs. control respectively. Results are representative of n = 3 experiments in triplicate. Study 2 - Biological/nutraceutical composition in combination with a diet enriched in palmitic acid for producing PEA *on demand* for the use in the treatment of functional gastrointestinal disorders (dyspepsia and irritable bowel syndrome).

Functional gastrointestinal disorders are high prevalence diseases in the population and are characterized by chronic pain in the absence of significant biochemical and/or clinical alterations explaining the origin of the symptoms.

The experimental study is aimed to evaluate the effectiveness of the LP-^{NAPE-PLD+} with the addition of palmitic acid, as a producer of PEA, in duodenal cultures from patients with functional dyspepsia. In duodenum of these subjects a modest mucosal inflammation with an increase in the number and activation of mast cells was observed, such events were associated with the presence of chronic pain and digestive disorders in patients.

Duodenal mucosal biopsies taken in the course of esophagogastroduodenoscopy from dyspeptic patients, defined by means of standardized clinical criteria known to experts in the field, were incubated in an acidic environment (pH = 3) such that to stimulate the activation of mucosal mast cells. The efficacy of the LP-^{NAPE-PLD+} (10¹⁰ cfu) in the presence of palmitic acid supplement (0.003 to 0.03 g/ml) in the culture medium was evaluated by counting the number of mast cells and measuring the release of specific mediators (histamine and tryptase). In addition, it was evaluated the expression of nerve fibers expressing the TRPV1 and TRPV4 type vanilloid receptors, which are associated to neurogenic inflammation and algogenic sensitivity. Taking in account that PEA is able to normalize the events above mentioned, it is expected that LP-^{NAPE-PLD+} in combination with palmitic acid, as a producer of high local concentrations of PEA, is able to normalize the processes acid-induced on mast cell and the TRPV1-4 activation.

### Experimental method

Cultures derived from full-thickness sections (whole mount) of duodenum from human subjects diagnosed with functional dyspepsia were used. The duodenal biopsies were cultured in Dulbecco's Modified Eagle Medium (DMEM) with FBS at 37 °C in 5% CO₂ atmosphere. Depending on the experimental protocol, the *in vitro* response in dyspeptic biopsy sampling, was triggered through stimulation at pH = 3; such stimulation was performed in the presence/absence of (10¹⁰ cfu) of LP-^{NAPE-PLD+} or LP^{empty vector} in the presence or absence of palmitic acid (0.003-0.03 µg/ml). Thirty minutes after the stimulus, levels of tryptase and histamine release were measured by ELISA. At the end of the treatment, biopsies were then homogenized and analyzed by Western blot. In the same experimental conditions, some samples were fixed in 4% paraformaldehyde (PFA) for subsequent histological analysis by toluidine bleu (0.05%) to assess the mast cell infiltration.

### Histological analysis

Sections 3-5 µm thick deriving from human duodenal biopsies were fixed in 4% PFA, and cut by cryostat. The sections were stained with toluidine blue 0.5% according to the standard manufacturer's protocol (Raymond Lamb Thermo Scientific, Fisher Scientific, UK).

### ELISA (enzyme-linked-immunosorbent assay) for histamine and tryptase release

The immunoassays (ELISA) for tryptase (Abcam, Cambridge, UK) and histamine (R&D Systems, Minneapolis, Minnesota, USA) were performed according to the protocol provided by the respective manufacturers. The absorbance was measured using a microplate reader Titertek (BioRad, Milan, Italy) and the histamine and tryptase levels were quantified by reference to the specific standard curves.

### Protein extraction and western blot analysis.

Biopsy samples were homogenized on ice with hypotonic lysis buffer to obtain cytosolic extracts. The extracts were subjected to electrophoresis through a 12% polyacrylamide gel. The proteins were transferred onto nitrocellulose membrane saturated with no-fat dry milk and then incubated with an anti-rabbit TRPV 1 antibody (Santa Cruz Biotech, California, USA; VR1 antibody H-150), anti-rabbit TRPV 4 (SantaCruz Biotech, California, USA; VR1 antibody H-150), anti-mouse β-actin (Santa Cruz Biotechnology, Santa Cruz, California, USA). The membranes were incubated with specific antibodies conjugated with horseradish peroxidase (HRP) (Dako, Milan, Italy). The immunocomplexes were detected by chemiluminescence (Amersham Biosciences, Milan, Italy). The blots were analyzed by scanning densitometry (GS-700 Imaging densitometer; Bio-Rad). The results were expressed as OD (arbitrary units per mm²) and normalized according to the expression of the β-actin.

The obtained results have showed that the association LP ^{NAPE-PLD+}/palmitate 0,003-0,03 µg/ml determines:
1. marked reduction in the number of mast cells in the duodenum mucosa of dyspeptic patient during acid stimulation *in vitro;*
2. reduced levels of released histamine and tryptase in the same cultured biopsies in the presence of acid stimulation *in vitro*;
3. significant reduction of TRPV1 and TRPV4 expression in tissue homogenates in the same biopsy samples under the same experimental conditions.

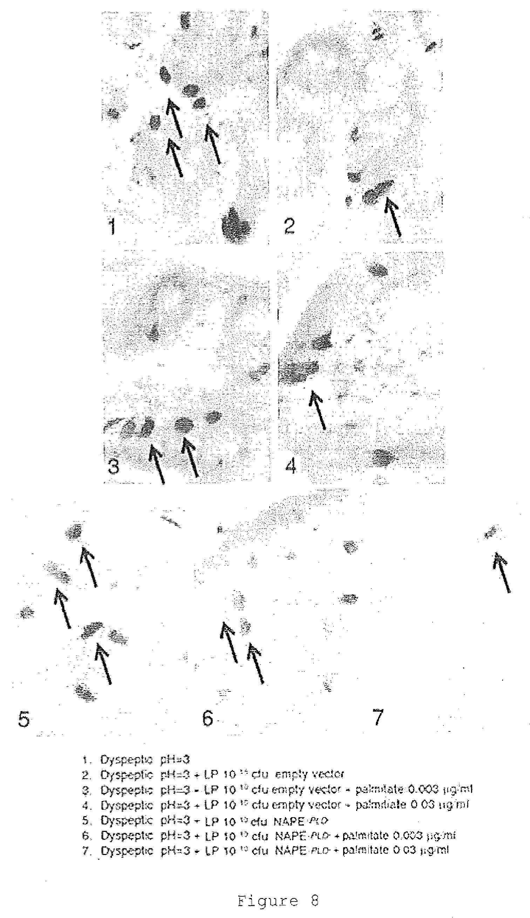

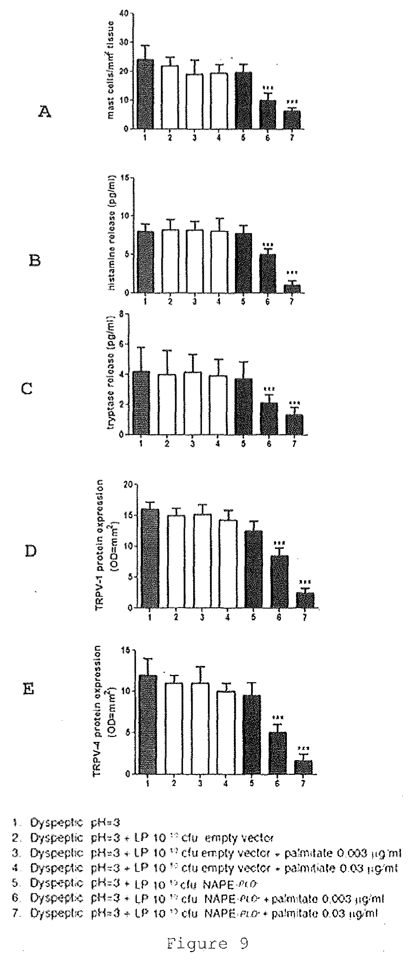

The results are shown in Figure 8 wherein it can be appreciated the effect of the daily administration of LP^{NAPE-PLD+} or LP ^{empty vector}, in the presence or absence of palmitate co-administration (0.03 to 0.3 mg/ml), in duodenal biopsies from dyspeptic patients and exposed to acid stimulation to pH = 3. The arrows indicate the mast cells colonizing the duodenal mucosal crypts detected by staining with toluidine blue (40X magnification). Figure 9, respectively, illustrates the number of mast cells (Fig. 9A), the histamine release (Fig. 9B), the level of tryptase (Fig. 9C), the TRPV1 (Fig. 9D) and TRPV4 (Fig. 9E) expression level. Results are representative of n = 3 experiments in triplicate. ***P<0.001 vs. dyspeptic exposed to acid stimulation.

Similarly, the therapeutic implications potentially due to LP^{NAPE-PLD+} in association with palmitic acid, as a producer of PEA, have been tested in patients with irritable bowel syndrome (IBS). In this syndrome are present predominantly characteristic disorders which include abdominal pain associated with the presence of constipation or diarrhea. The complex patho-physiologic mechanisms basing IBS, are not well understood but include altered intestinal motility, visceral hypersensitivity and more recently microscopic inflammation of the colon. Experimental studies have established that the PEA, having analgesic and anti-inflammatory action, as described in detail above, is able to determine a significant symptomatic improvement in this type of functional disorder.

Therefore, it was assessed the impact of the LP-^{NAPE-PLD+} in combination of palmitic acid (0.003-0.03 µg/ml), in colon biopsies of IBS patients, by evaluating the effectiveness of the administration on histopathology in terms jof mast cell infiltration and by measuring tryptase and histamine release. Preliminary data from the trial are very encouraging. Further and more in-depth analysis to ascertain the effectiveness of the association of LP ^{NAPE-PLD+} with palmitic acid in the feeding are currently on going.

Study 3 - LP-^{NAPE-PLD+} in association with oleic acid for on-demand production of OEA for the use in the treatment of obesity and related diseases (cardiovascular disease, high cholesterol, diabetes, metabolic syndrome).

The effectiveness of the administration of LP-^{NAPE-PLD+} (10¹⁰ cfu) was evaluated by observing the amount of food intake and feeding behavior in ob/ob mice in the presence and in the absence of a diet based on oleic acid (0.003 to 0.03 mg/ml/day).

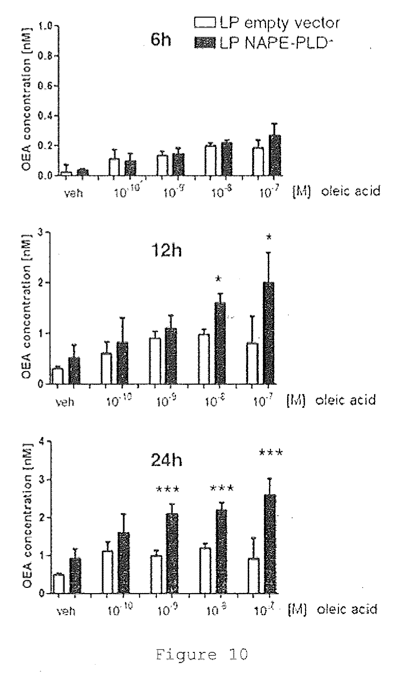

The test has been carried out for 21 days and the animals were periodically weighed and their food and water consumption monitored by suitable cages BioDAQ-Standard Research Diet Inc. (New Brunswick, NJ, USA), which provides for accurate measurement of food intake of each individual test animal. At different time intervals blood samples of animals were collected and the OEA levels were obtained using the experimental protocol described above. Further confirmation *in vitro* on human biopsies of the small intestine, grown, following incubation with LP^{NAPE-PLD+} in the presence and in the absence of oleic acid (0.003 to 0.03 **µ**g/ml/day), have provided an estimate of the released OEA (Fig. 10).

**Study 4 -** Impact assessment of LP-^{NAPE-PLD+} in combination with oleic acid on total metabolic pattern in genetically obese mice.

The ob/ob mice have obese phenotype with many complications reflecting in general terms the features of the human metabolic syndrome, such as hyperphagia, altered sense of satiety, hypercholesterolemia, hyperinsulinemia, hyperglycemia, non-linear increase in growth, hypothermia, low consumption of energy and oxygen, increase in glucocorticoids concentration, hypogonadism and abnormalities in the immune function. In ob/ob mice the effect of the administration of LP-^{NAPE-PLD+} (10¹⁰ cfu) associated or not to a diet based on oleic acid (0.003 to 0.03 g/ml/day) for a period of 4 weeks was evaluated. At the end of the 4-week treatment the values of blood pressure, the energy requirements and laboratory parameters (AST, ALT, cholesterol, insulin, glycosylated hemoglobin, triglycerides, C-reactive protein, etc.) were evaluated, as an index of a any improvement in the total metabolic pattern.

### Experimental method

In order to quantify the amount of OEA *in vitro* the supernatant from cultures of LP^{NAPE-PLD+} or LP^{empty vector} in the presence of external support of oleic acid (10⁻¹⁰-10⁻⁷ M) was analyzed after 6-12-24 hours by chromatography liquid coupled to mass spectrometry (LC-MS/MS) using a mass spectrometer 325 triple quadrupole LC-MS/MS (Agilent Technologies Italy, g biological composition containing 10¹⁰ cfu of LP-^{NAPE-PLD+} by gavage, the third grou received a daily intake via gastric gavage of biological composition containing 10¹⁰ cfu of LP-^{NAPE-PLD+} in association with oiled (0.03 mg/ml). The animal weight was determined every 24 hours and the food intake of each individual test animal was measured through the use of suitable cages BioDAQ-Standard Research Diet Inc. (New Brunswick, NJ, USA) according to the manufacturer's instructions.

### Cholesterol and triglycerides dosage

At the end of the experimental protocol the levels of total cholesterol and triglycerides were assessed in accordance with the procedures of diagnostic test kit MULTICARE, supplied by the manufacturer (Beinasco, Turin, Italy).

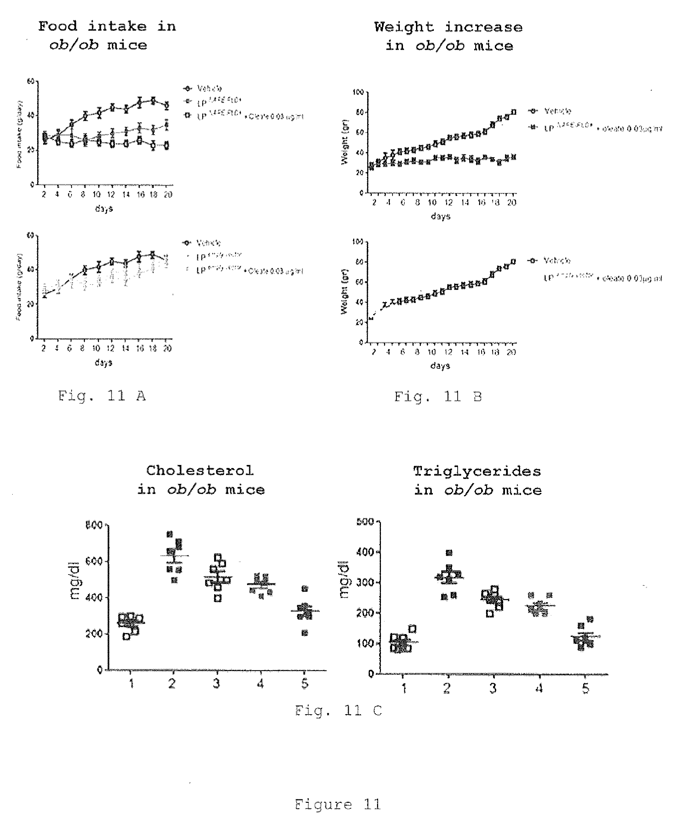

Preliminary results have shown that *in vitro* under exogenous drive of oleic acid administration a progressive and time-dependent increase in the release of OEA in the culture medium of the bacterial over 6-24 hours occurs. This increase is accompanied by other effects observed in ob/ob mice showing that the association of the probiotic LP^{NAPE-PLD+} and 0.03 mg/ml oleate administered for 21 days by gastric gavage, causes a progressive reduction of food intake and weight gain (Fig. 11A and B) and an increase of cholesterol and triglycerides control compared to control animals (Fig. 11C). These beneficial effects are not observed in the case of a daily administration of LP^{empty vector} even in the presence of a similar support with oleate. These results, although preliminary, demonstrate the considerable potential of the invention in terms of controlling obesity and metabolic syndrome.

### Conclusions

From the above description is therefore evident that the characterizing and innovative aspect of the present invention primarily resides in the use of a particular probiotic bacterial strain, the L. Paracasei F19, which unlike those used in the prior art for the transformation with the gene coding NAPE-PLD (PEA and OEA), such as bacteria of the species Bacillus, Escherichium, Bifidobacterium, Lactobacillus, Lactococcus, Enterobacteriaceae, and Saccharomyces (as in WO 2013/043719), is a strain genetically stable. But moreover, the innovative aspect which cannot be derived in any way from the teachings of the prior art is the association of the use of probiotic genetically modified to produce increased levels of pharmacologically active lipids ALIA-mides (PEA and OAS), with the conditioning of the metabolism of the probiotic organism itself through specific and targeted dietary supplement aimed to produce OEA or PEA on the real requirements of the individual. Thus, the technical solution proposed by the invention is to drive the microbiota engineered to produce what is specifically therapeutically required depending on the condition for which the method will be applied to, such as obesity, diabetes, cardiovascular disease or IBS, abdominal pain, inflammation of the bowel.

### REFERENCES

1. Grozdanov L. et al. Analysis of the genome structure of the nonpathogenic probiotic Escherichia coli strain Nissle 1917. J Bacteriol 2004; 186:5432- 41.
2. Grozdanov L. et al. A single nucleotide exchange in the wzy gene is responsible for the semirough 06 lipopolysaccharide phenotype and serum sensitivity of Escherichia coli strain Nissle 1917. J Bacteriol 2002; 184:5912-25.
3. Nougayrède J.P. et al. Escherichia coli induces DNA double-strand breaks in eukaryotic cells. Science 2006; 313:848-51.
4. Cuevas-Ramos G. et al. Escherichia coli induces DNA damage in vivo and triggers genomic instability in mammalian cells. Proc Natl Acad Sci U S A 2010; 107:11537-42.
5. Morelli L. et al. Genetic stability of Lactobacillus paracasei subsp. paracasei F19. (2002) Microbial Ecology in Health and Disease Suppl 3: 14-16.
6. Annibale B. et al. Flora intestinale, antibiotici, probiotici, nutrigenomici. (2005) Ospedale & Territorio - Gastroenterologia. 6: 47-58.

## Claims

1. A method for modulating the production of active lipids involved in exerting an antagonistic action to local inflammation and in modulating the mast cell reactivity, comprising administering to an host the probiotic *Lactobacillus paracasei* subsp. Paracasei F19, genetically modified with a nucleotide vector comprising a nucleic acid encoding the human N-acyl-phosphatidylethanolamine phospholipase D (NAPE-PLD) of SEQ ID NO: 1, in association with palmitic acid or oleic acid, or the salts thereof, to influence the enzymatic reactions of said probiotic agent in the host.

2. Method according to claim 1 wherein the active lipids involved in exerting an antagonistic action to local inflammation and in modulating the mast cell reactivity are palmitoylethanolamide (PEA) and oleoylethanolamide (OEA).

3. *Lactobacillus paracasei* subsp paracasei F19 transformed with a nucleotide vector comprising a nucleic acid encoding the human N-acyl-phosphatidylethanolamine phospholipase D (NAPE-PLD) of SEQ ID NO: 1.

4. A composition comprising:
- *Lactobacillus paracasei* subsp Paracasei F19 overexpressing N-acyl phosphatidylethanolamine phospholipase D (NAPE-PLD),
- saturated and/or unsaturated fatty acids, or salts thereof,
- an ingestible vehicle suitable for oral administration of the composition.

5. The composition according to claim 4 further comprising dietary fiber, maltodextrin, glucoligosaccharides, fructooligosaccharides, prebiotic agents, pectin, inulin, amino acids, mineral salts, vitamins, soy protein, corn protein, antioxidant agents.

6. The composition according to claims 4 and 5 wherein *Lactobacillus paracasei* subsp paracasei F19 is transformed with a nucleotide vector comprising the nucleic acid encoding the human N-acyl-phosphatidylethanolamine phospholipase D (NAPE-PLD) of sequence SEQ. ID NO: 1.

7. The composition according to any claims from 4 to 6 wherein the transformed *Lactobacillus paracasei* subsp paracasei F19 is in lyophilized form in an amount ranging between 1x10⁹ and 1x10¹¹ cfu per milligram of the composition.

8. The composition according to any claims from 4 to 7 wherein the fatty acids are present in an amount ranging between 3 and 9 mg per 100 mg of composition.

9. The composition according to claim 8 wherein the fatty acids are palmitic or oleic acid.

10. Composition according to any claims from 4 to 10 provided in an edible product.

11. Composition according to claim 11, wherein the edible product is a dietary bar, beverage, juice, milk, functional beverage, beverage comprising dietary fiber, food, yogurt, candy, gum, capsules, granular powder, tablets.

12. A composition comprising:
- *Lactobacillus paracasei* subsp Paracasei F19 overexpressing N-acyl phosphatidylethanolamine phospholipase D (NAPE-PLD),
- palmitic or oleic acid, or the salts thereof,
- an ingestible vehicle edible suitable for the oral administration of the composition,
for the use as a medicament.

13. A composition comprising:
- *Lactobacillus paracasei* subsp Paracasei F19 overexpressing N-acyl phosphatidylethanolamine phospholipase D (NAPE-PLD),
- palmitic acid, or its salts,
- an ingestible vehicle suitable for oral administration of the composition,
for the use in the treatment of inflammatory bowel disorder.

14. A composition comprising:
- *Lactobacillus paracasei* subsp Paracasei F19 overexpressing N-acyl phosphatidylethanolamine phospholipase D (NAPE-PLD),
- Oleic acid or its salts,
- an ingestible vehicle suitable for oral administration of the composition,
for the use in the treatment of metabolic syndrome and for controlling of obesity.
